# FASCICULE DE BREVET EUROPEEN

(11) **EP 1 106 207 B1**
(45) Date de publication et mention de la délivrance du brevet: **26.07.2006**
(21) Numéro de dépôt: 00403384.1
(22) Date de dépôt: 01.12.2000
(51) Int. Cl.: A61N 1/368, A61N 1/39

(54) **Dispositif médical implantable actif, notamment stimulateur cardiaque, défibrillateur et/ou Cardiovecteur de type multisite, comportant des moyens de détection des tachycardies induites**
Aktive implantierbare medizinische Vorrichtung, insbesondere mehrstelliger Herzstimulator, Defibrillator und/oder Kardiovertierer, mit Mitteln zur Detektion induzierter Tachykardien
Active implantable medical device, in particular multisite pacemaker, defibrillator and/or cardioverter, having means for detecting induced tachycardias

(30) Priorité: 02.12.1999 FR 9915220
(43) Date de publication de la demande: 13.06.2001
(73) Titulaire: ELA MEDICAL (Société anonyme), 92541 Montrouge (FR)
(72) Inventeur: Ripart, Alain, 91190 Gif sur Yvette (FR)
(74) Mandataire: Dupuis-Latour, Dominique

(56) Documents cités:
- EP-A- 0 676 216
- EP-A- 0 813 889
- EP-A- 0 862 927
- EP-A- 0 935 979
- US-A- 5 983 138

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement les prothèses dites "multisite", dans lesquelles des électrodes sont placées en une pluralité de sites respectifs distincts. A la différence des stimulateurs "double chambre" qui ne comportent qu'un site ventriculaire et un site auriculaire (cf. le US-A-5 983 138), les dispositifs "multisite" comportent au moins deux sites ventriculaires et/ou deux sites auriculaires. Les EP-A-0 676 216, EP-A-0 813 889, EP-A-0 862 927 et EP-A-0 935 979 décrivent de telles prothèses "triple chambre" (stimulation atriale droite et double stimulation ventriculaire) ou "quadruple chambre" (double stimulation atriale et double stimulation ventriculaire). Il peut également s'agir d'une prothèse permettant la stimulation d'une même cavité en deux sites distincts, par exemple une double stimulation du ventricule gauche.

En effet, outre le traitement des troubles du rythme cardiaque, il a été proposé de traiter par stimulation les troubles de la contraction myocardique observés chez des patients en insuffisance cardiaque, ces troubles pouvant être spontanés ou induits par une stimulation traditionnelle. On pourra notamment se référer à l'étude de J. C. Daubert et coll., *Stimucoeur,* 25, n°3, pp. 170-176 qui fait le point des travaux sur ce sujet.

Il a été notamment proposé de stimuler simultanément les ventricules gauche et droit, en permanence, pour les resynchroniser, ce qui a permis d'observer des résultats souvent spectaculaires pour des patients en insuffisance cardiaque en classe III, non améliorée par les traitements classiques.

Par la suite, on décrira le cas d'une stimulation des cavités basses, c'est-à-dire le cas d'une double stimulation ventriculaire, car ce cas est celui qui est le plus défavorable pour la fonction cardiaque du patient ; le mécanisme que l'on va décrire peut cependant affecter de la même manière les cavités hautes, et les solutions proposées pourront être appliquées *mutatis mutandis* à une double stimulation auriculaire.

Après délivrance d'une impulsion électrique de stimulation, un front de dépolarisation se propage dans le volume du myocarde autour du point de stimulation, avec pour conséquence la création après le passage de ce front d'une période réfractaire (de l'ordre de 250 ms) durant laquelle les cellules cardiaques ne sont plus excitables. Cette période réfractaire est suivie, avant retour à l'état normal, par une période transitoire durant laquelle les cellules cardiaques sont, au contraire, hyperexcitables de sorte que toute stimulation tombant dans cette dernière période, qu'elle soit d'origine naturelle ou stimulée, peut à nouveau redéclencher l'état d'excitation d'une cellule cardiaque et engendrer ainsi un phénomène de tachycardie indésirable, c'est-à-dire une fréquence anormalement élevée, du fait de l'instabilité électrique des cellules.

Un autre phénomène propre au fonctionnement des dispositifs multisite tient au fait que la stimulation simultanée, ou avec un léger décalage, de deux ou plusieurs sites peut conduire à la création de zones présentant des aberrations de la conduction dans la zone de rencontre des deux fronts de propagation de la dépolarisation. Ceci peut conduire à l'apparition d'une zone dite de bloc dans lequel la propagation sera ralentie, voire stoppée.

Ainsi, la stimulation concomitante des deux ventricules peut entraîner la formation d'une zone de bloc dans la région interventriculaire, qui est celle de la transmission atrioventriculaire.

Par ailleurs, une double stimulation auriculaire peut engendrer le phénomène suivant : la première stimulation va entraîner, par conduction normale, la stimulation subséquente du ventricule ; la seconde stimulation, dont la propagation sera retardée dans la zone de bloc, va également atteindre le ventricule mais, du fait du retard subi, va l'atteindre à une période où celui-ci n'est plus dans sa période réfractaire, et va donc provoquer une contraction indésirable entraînant, ici encore, un trouble du rythme pouvant évoluer en crise de tachycardie.

L'un des buts de l'invention est de remédier à ces divers phénomènes dits de "tachycardie induite" ou "tachycardie par ré-entrée" (TRE ou PMT, *Pacemaker-Mediated Tachycardia)* qui peuvent être causées par les stimulateurs multisite.

À cet effet, l'invention propose un dispositif du type multisite tel que par exemple décrit dans le EP-A-0 676 216 précité, c'est-à-dire dans lequel des électrodes sont adaptées pour être placées en au moins deux sites ventriculaires, droit et gauche, et/ou en au moins deux sites auriculaires, droit et gauche ces électrodes étant reliées à un circuit de recueil de signaux cardiaques pour détecter un potentiel de dépolarisation sur au moins l'un des sites, ainsi qu'à un circuit de stimulation pour appliquer des impulsions de stimulation sur au moins certains desdits sites.

Selon l'invention, le dispositif comporte des moyens de suivi de la fréquence cardiaque, des moyens de détection de tachycardie induite, fonctionnant en réponse aux moyens de suivi pour décider la présence d'une tachycardie induite si la fréquence cardiaque dépasse un seuil prédéterminé pendant une durée supérieure à un seuil prédéterminé, et des moyens pour modifier temporairement la stimulation simultanée par le dispositif en cas de tachycardie induite détectée.

De préférence, les moyens de détection ne décident la présence d'une tachycardie induite que si le taux d'accroissement de la fréquence cardiaque dépasse une valeur minimale prédéterminée.

Les moyens pour modifier le fonctionnement du dispositif peuvent avantageusement être des moyens aptes à réduire le délai de décalage de stimulation entre les deux sites ventriculaires droit et gauche, et/ou entre les deux sites auriculaires droit et gauche.

Ces moyens peuvent également être, en variante ou en complément, des moyens aptes à inhiber la stimulation de l'un des deux sites ventriculaires droit et gauche, et/ou de l'un des deux sites auriculaires droit et gauche, et/ou de l'un des deux sites d'une même cavité. Avantageusement, on peut prévoir que ces moyens ne soient activés qu'après activation préalable des moyens aptes à réduire le délai de décalage de stimulation et persistance consécutive de la tachycardie.

Après activation des moyens pour modifier le fonctionnement du dispositif, ceux-ci peuvent être maintenus activés pendant une durée prédéterminée, puis désactivés. Avantageusement, on peut prévoir qu'après désactivation de ces moyens, ceux-ci sont réactivés de façon permanente en cas de nouvelle détection de tachycardie induite.

On va maintenant décrire un exemple de réalisation du dispositif de l'invention.

Celle-ci est mise en oeuvre dans un dispositif tel que celui décrit dans le EP-A-0 925 806 (ELA Médical), auquel on se référera pour de plus amples détails, l'invention résultant d'une programmation appropriée du logiciel de pilotage du dispositif multisite.

Le dispositif assure le suivi continu de la fréquence cardiaque.

Si cette fréquence dépasse un certain seuil (par exemple 120 cpm), le dispositif entre dans une phase de suspicion de tachycardie induite.

Un critère supplémentaire pour avérer cette suspicion consiste à évaluer le taux d'accroissement de la fréquence : si la fréquence s'accroît relativement lentement, il peut s'agit d'une variation physiologique, à l'effort par exemple ; en revanche, si l'augmentation de fréquence est soudaine, l'hypothèse d'une tachycardie induite est plus plausible.

Après la phase de suspicion, le dispositif entre dans une phase de confirmation de tachycardie induite : il y a confirmation si la fréquence cardiaque enregistrée se maintient au-dessus du seuil considéré pendant un intervalle de temps prédéterminé programmable, par exemple pendant plus d'une minute. Si tel n'est pas le cas, c'est-à-dire si la fréquence redescend au-dessous de la valeur de seuil, le dispositif n'entreprend aucune modification de son fonctionnement, et continue seulement le suivi de la fréquence cardiaque, dans l'attente d'une nouvelle suspicion de tachycardie induite.

En cas de tachycardie induite confirmée, le dispositif modifie son fonctionnement de manière à essayer de faire disparaître cette tachycardie induite.

Dans un premier stade, si l'on est dans un mode de stimulation simultané, le dispositif tente de réduire le décalage de stimulation entre les deux ventricules, ou entre les deux oreillettes, ou entre les sites d'une même cavité si celle-ci est stimulée en plusieurs points.

Si la tachycardie induite persiste en dépit de cette réduction du décalage entre les stimulations, le dispositif inhibe la stimulation sur certains des sites : le dispositif peut par exemple inhiber la stimulation du ventricule gauche dans le cas d'une stimulation ventriculaire, ou inhiber la stimulation sur l'un des sites si une même cavité est stimulée en plusieurs points

Si la tachycardie induite cesse après ces modifications de fonctionnement, on peut avantageusement prévoir que le dispositif fasse une nouvelle tentative de stimulation multisite ultérieurement, par exemple après plusieurs jours passés dans la configuration modifiée. En cas de réapparition du phénomène, le dispositif maintiendra la configuration modifiée jusqu'à intervention ultérieure d'un clinicien.

Ces différents événements, ainsi que leur moment d'apparition, peuvent bien entendu être enregistrés dans la mémoire du dispositif, selon des modalités connues, pour consultation ultérieure.

## Revendications

1. Un dispositif médical implantable actif, notamment un stimulateur cardiaque, défibrillateur et/ou cardioverteur,
ce dispositif étant un dispositif du type multisite relié à des électrode adaptées pour être placées en au moins deux sites ventriculaires et/ou en au moins deux sites auriculaires, et comprenant :
- des moyens de stimulation ventriculaire double, aptes à délivrer simultanément des impulsions de stimulation auxdits deux sites ventriculaires pour les resynchroniser, et/ou
- des moyens de stimulation auriculaire double, aptes à délivrer simultanément des impulsions de stimulation auxdits deux sites auriculaires pour les resynchroniser, et
- un circuit de recueil de signaux cardiaques, apte à détecter un potentiel de dépolarisation sur au moins l'un desdits sites,
dispositif **caractérisé en ce qu'**il comporte en outre :
- des moyens de suivi de la fréquence cardiaque,
- des moyens de détection de tachycardie induite, fonctionnant en réponse aux moyens de suivi pour décider la présence d'une tachycardie induite si la fréquence cardiaque dépasse un seuil prédéterminé pendant une durée supérieure à un seuil prédéterminé, et
- des moyens pour modifier temporairement la stimulation simultanée par le dispositif en cas de tachycardie induite détectée.

2. Le dispositif de la revendication 1, dans lequel les moyens de détection sont adaptés pour ne décider la présence d'une tachycardie induite que si le taux d'accroissement de la fréquence cardiaque dépasse une valeur minimale prédéterminée.

3. Le dispositif de la revendication 1, dans lequel les moyens pour modifier le fonctionnement du dispositif sont des moyens aptes à réduire le délai de décalage de stimulation entre les deux sites ventriculaires droit et gauche, et/ou entre les deux sites auriculaires droit et gauche.

4. Le dispositif de la revendication 1, dans lequel les moyens pour modifier le fonctionnement du dispositif sont des moyens aptes à inhiber la stimulation de l'un des deux sites ventriculaires droit et gauche, et/ou de l'un des deux sites auriculaires droit et gauche.

5. Le dispositif des revendications 3 et 4 prises en combinaison, dans lequel les moyens aptes à inhiber la stimulation de l'un des deux sites sont adaptés pour ne être activés qu'après activation préalable des moyens aptes à réduire le délai de décalage de stimulation et persistance consécutive de la tachycardie.

6. Le dispositif de la revendication 1, dans lequel, les moyens pour modifier temporairement la stimulation simultanée par le dispositif, sont adaptés pour que, après leur activation, ceux-ci sont maintenus activés pendant une durée prédéterminée, puis désactivés.

7. Le dispositif de la revendication 6, dans lequel, les moyens pour modifier temporairement la stimulation simultanée par le dispositif sont adaptés pour que, après leur desactivation, ceux-ci sont réactivés de façon permanente en cas de nouvelle détection de tachycardie induite.

## Claims

1. An active implantable medical device, in particular pacemaker, defibrillator and/or cardioverter, said device being a device of the multisite type, connected to electrodes adapted to be placed in at least two ventricular sites and/or at least two atrial sites, comprising:
- double ventricular stimulation means, being able to deliver stimulation pulses simultaneously to said two ventricular sites in order to resynchronise them, and/or
- double atrial stimulation means, being able to deliver stimulation pulses simultaneously to said two atrial sites in order to resynchronise them, and
- a cardiac signal collection circuit, being able to detect a depolarization potential on at least one of said sites,
a device **characterised in that** it further comprises:
- means for monitoring the heart rate,
- means for detecting an induced tachycardia, working in response to the monitoring means to decide about the presence of an induced tachycardia if the heart rate exceeds a predetermined threshold for a length of time greater than a predetermined threshold, and
- means for temporarily modifying the simultaneous stimulation by the device in case of a detected induced tachycardia.

2. The device of claim 1, wherein the detecting means is adapted to decide on the presence of an induced tachycardia only when the rate of increase of the heart rate exceeds a predetermined minimum value.

3. The device of claim 1, wherein the means for modifying the device's operation is a means being able to reduce the time span of the stimulation offset between the two ventricular sites, right and left, and/or the two atrial sites, right and left.

4. The device of claim 1, wherein the means for modifying the device's operation is a means being able to inhibit the stimulation of one of said two ventricular sites, right and left, and/or of one of said two atrial sites, right and left.

5. The device of claims 3 and 4 in combination, wherein the means able to inhibit the stimulation of one of said two sites is adapted to be activated only after previous activation of the means able to reduce the time span of the stimulation offset and consecutive persistence of the tachycardia.

6. The device of claim 1, wherein the means for temporarily modifying the simultaneous stimulation by the device is adapted so that, after its activation, it is maintained activated during a predetermined duration, and then deactivated.

7. The device of claim 6, wherein the means for temporarily modifying the simultaneous stimulation by the device is adapted so that, after its deactivation, it is reactivated in a permanent manner in case of a new detection of an induced tachycardia.

## Patentansprüche

1. Aktive implantierbare medizinische Vorrichtung, insbesondere Herzschrittmacher, Defibrillator, und/oder Kardioverter,
wobei diese Vorrichtung eine Vorrichtung vom Multisite-Typ ist, der mit Elektroden verbunden ist, die an wenigstens zwei Herzkammersitzen und/oder an wenigstens zwei Herzvorhofsitzen platzierbar sind, mit:
- doppelten Mitteln zur Herzkammerstimulation, die dazu geeignet sind, Stimulationsimpulse gleichzeitig an die zwei Herzkammersitze abzugeben, um sie wieder zu synchronisieren, und/oder,
- doppelten Mitteln zur Herzvorhofstimulation, die dazu geeignet sind, Stimulationsimpulse gleichzeitig an die zwei Herzvorhofsitze abzugeben, um sie wieder zu synchronisieren, und
- einer Schaltung zur Aufnahme von Herzsignalen, die dazu geeignet ist, ein Depolarisationspotential an wenigstens einem der Sitze zu erfassen,
Vorrichtung, die **dadurch gekennzeichnet ist, dass** sie außerdem folgendes umfasst:
- Mittel zur Verfolgung der Herzfrequenz,
- Mittel zur Erfassung einer induzierten Tachykardie, welche in Antwort auf die Verfolgungsmittel arbeiten, um über das Vorliegen einer induzierten Tachykardie zu entscheiden, wenn die Herzfrequenz eine vorbestimmte Schwelle über eine Dauer überschreitet, die größer ist als eine vorbestimmte Schwelle, und
- Mitteln zur zeitweisen Änderung der gleichzeitigen Stimulation durch die Vorrichtung im Falle einer erfassten induzierten Tachykardie.

2. Vorrichtung nach Anspruch 1, bei welcher die Erfassungsmittel dafür ausgelegt sind, das Vorliegen einer induzierten Tachykardie nur dann zu cntscheiden, wenn die Steigerungsrate der Herzfrequenz einen minimalen vorbestimmten Wert überscbreitet.

3. Vorrichtung nach Anspruch 1, bei welcher die Mittel zur Änderung des Betriebs der Vorrichtung Mittel sind, die dazu geeignet sind, den Zeitraum der Verschiebung der Stimulation zwischen den zwei Herzkammersitzen, rechts und links, und/oder zwischen den zwei Herzvorhofsitzen, rechts und links, zu verringern.

4. Vorrichtung nach Anspruch 1, bei welcher die Mittel zur Änderung des Betriebs der Vorrichtung Mittel sind, die dazu geeignet sind, die Stimulation eines der zwei Herzkammersitze, rechts und links, und/oder eines der zwei Herzvorhofsitze, rechts und links, zu hemmen.

5. Vorrichtung nach den Ansprüchen 3 und 4 in Kombination, bei welcher die Mittel, die geeignet sind, die Stimulation eines der zwei Sitze zu hemmen, dafür ausgelegt sind, nur nach vorheriger Aktivierung der Mittel zur Verringerung des Zeitraums der Verschiebung der Stimulation und darauf folgendes Fortdauern der Tachykardie aktiviert zu werden.

6. Vorrichtung nach Anspruch 1, bei welcher die Mittel zur zeitweisen Änderung der gleichzeitigen Stimulation durch die Vorrichtung dafür ausgelegt sind, dass, nach deren Aktivierung, diese über eine vorbestimmte Dauer aktiviert bleiben und sodann deaktiviert werden.

7. Vorrichtung nach Anspruch 6, bei welcher die Mittel zur zeitweisen Änderung der gleichzeitigen Stimulation durch die Vorrichtung dafür ausgelegt sind, dass, nach deren Deaktivierung, diese im Falle einer erneuten Erfassung einer induzierten Tachykardie auf permanente Weise reaktiviert werden.
